# EUROPEAN PATENT APPLICATION

(11) **EP 0 860 700 A2**
(43) Date of publication of application: **26.08.1998**
(21) Application number: 98301123.0
(22) Date of filing: 16.02.1998
(51) Int. Cl.: G01N 33/50, G01N 33/569

(54) **Use of HSV-1 UL-15 and VP5 in identifying anti-viral agents**

(30) Priority: 21.02.1997 US 38914 P
(71) Applicant: SMITHKLINE BEECHAM CORPORATION, Philadelphia Pennsylvania 19103 (US)
(72) Inventor: Del Vecchio, Alfred, King of Prussia, PA 19406 (US)
(74) Representative: Crump, Julian Richard John

(57) **Abstract**

The present invention provides a method for identifying compounds having antiviral activity against members of the herpes virus family. This method involves the identification of compounds which inhibit the interaction between the gene products of the herpes simplex virus-1 UL15 gene and the UL19 gene (VP5). Also provided are compounds identified by the method of the invention.

### Annex to the description

SEQUENCE LISTING

(1) GENERAL INFORMATION:

(i) APPLICANT: SmithKline Beecham Corporation

(ii) TITLE OF INVENTION: Use of HSV-1 UL-15 and VP5 in
Identifying Anti-Viral Agents

(iii) NUMBER OF SEQUENCES: 2

(iv) CORRESPONDENCE ADDRESS:
(A) ADDRESSEE: F J Cleveland & Company
(B) STREET: 40/43 Chancery Lane
(C) CITY: London
(D) COUNTY:
(E) COUNTRY: United Kingdom
(F) POST CODE: WC2A 1JQ

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: US
(B) FILING DATE:
(C) CLASSIFICATION:

(viii) ATTORNEY/AGENT INFORMATION:
(A) NAME: CRUMP, Julian Richard John
(B) GENERAL AUTHORISATION NUMBER: 37127
(C) REFERENCE/DOCKET NUMBER: P50617

(ix) TELECOMMUNICATION INFORMATION:
(A) TELEPHONE: +44 171 405 5875
(B) TELEFAX: +44 171 831 0749

(2) INFORMATION FOR SEQ ID NO: 1:

(i) SEQUENCE CHARACTERISTICS:
(A) LENGTH: 735 amino acids
(B) TYPE: amino acid
(C) STRANDEDNESS:
(D) TOPOLOGY: unknown

(ii) MOLECULE TYPE: protein

(xi) SEQUENCE DESCRIPTION: SEQ ID NO: 1:

Met Phe Gly Gln Gln Leu Ala Ser Asp Val Gln Gln Tyr Leu Glu Arg
1               5                   10                  15

Leu Glu Lys Gln Arg Gln Leu Lys Val Gly Ala Asp Glu Ala Ser Ala
            20                  25                  30

Gly Leu Thr Met Gly Gly Asp Ala Leu Arg Val Pro Phe Leu Asp Phe
        35                  40                  45

Ala Thr Ala Thr Pro Lys Arg His Gln Thr Val Val Pro Gly Val Gly
    50                  55                  60

Thr Leu His Asp Cys Cys Glu His Ser Pro Leu Phe Ser Ala Val Ala
65                  70                  75                  80

Arg Arg Leu Leu Phe Asn Ser Leu Val Pro Ala Gln Leu Lys Gly Arg
                85                  90                  95

Asp Phe Gly Gly Asp His Thr Ala Lys Leu Glu Phe Leu Ala Pro Glu
            100                 105                 110

Leu Val Arg Ala Val Ala Arg Leu Arg Phe Lys Glu Cys Ala Pro Ala
        115                 120                 125

Asp Val Val Pro Gln Arg Asn Ala Tyr Tyr Ser Val Leu Asn Thr Phe
    130                 135                 140

Gln Ala Leu His Arg Ser Glu Ala Phe Arg Gln Leu Val His Phe Val
145                 150                 155                 160

Arg Asp Phe Ala Gln Leu Leu Lys Thr Ser Phe Arg Ala Ser Ser Leu
                165                 170                 175

Thr Glu Thr Thr Gly Pro Pro Lys Lys Arg Ala Lys Val Asp Val Ala
            180                 185                 190

Thr His Gly Arg Thr Tyr Gly Thr Leu Glu Leu Phe Gln Lys Met Ile
        195                 200                 205

Leu Met His Ala Thr Tyr Phe Leu Ala Ala Val Leu Leu Gly Asp His
    210                 215                 220

Ala Glu Gln Val Asn Thr Phe Leu Arg Leu Val Phe Glu Ile Pro Leu
225                 230                 235                 240

Phe Ser Asp Ala Ala Val Arg His Phe Arg Gln Arg Ala Thr Val Phe
                245                 250                 255

Leu Val Pro Arg Arg His Gly Lys Thr Trp Phe Leu Val Pro Leu Ile
            260                 265                 270

Ala Leu Ser Leu Ala Ser Phe Arg Gly Ile Lys Ile Gly Tyr Thr Ala
        275                 280                 285

His Ile Arg Lys Ala Thr Glu Pro Val Phe Glu Glu Ile Asp Ala Cys

    290                 295                 300

Leu Arg Gly Trp Phe Gly Ser Ala Arg Val Asp His Val Lys Gly Glu
305                 310                 315                 320

Thr Ile Ser Phe Ser Phe Pro Asp Gly Ser Arg Ser Thr Ile Val Phe
                325                 330                 335

Ala Ser Ser His Asn Thr Asn Gly Ile Arg Gly Gln Asp Phe Asn Leu
            340                 345                 350

Leu Phe Val Asp Glu Ala Asn Phe Ile Arg Pro Asp Ala Val Gln Thr
        355                 360                 365

Ile Met Gly Phe Leu Asn Gln Ala Asn Cys Lys Ile Ile Phe Val Ser
    370                 375                 380

Ser Thr Asn Thr Gly Lys Ala Ser Thr Ser Phe Leu Tyr Asn Leu Arg
385                 390                 395                 400

Gly Ala Ala Asp Glu Leu Leu Asn Val Val Thr Tyr Ile Cys Asp Asp
                405                 410                 415

His Met Pro Arg Val Val Thr His Thr Asn Ala Thr Ala Cys Ser Cys
            420                 425                 430

Tyr Ile Leu Asn Lys Pro Val Phe Ile Thr Met Asp Gly Ala Val Arg
        435                 440                 445

Arg Thr Ala Asp Leu Phe Leu Ala Asp Ser Phe Met Gln Glu Ile Ile
    450                 455                 460

Gly Gly Gln Ala Arg Glu Thr Gly Asp Asp Arg Pro Val Leu Thr Lys
465                 470                 475                 480

Ser Ala Gly Glu Arg Phe Leu Leu Tyr Arg Pro Ser Thr Thr Thr Asn
                485                 490                 495

Ser Gly Leu Met Ala Pro Asp Leu Tyr Val Tyr Val Asp Pro Ala Phe
            500                 505                 510

Thr Ala Asn Thr Arg Ala Ser Gly Thr Gly Val Ala Val Val Gly Arg
        515                 520                 525

Tyr Arg Asp Asp Tyr Ile Ile Phe Ala Leu Glu His Phe Phe Leu Arg
    530                 535                 540

Ala Leu Thr Gly Ser Ala Pro Ala Asp Ile Ala Arg Cys Val Val His
545                 550                 555                 560

Ser Leu Thr Gln Val Leu Ala Leu His Pro Gly Ala Phe Arg Gly Val
                565                 570                 575

Arg Val Ala Val Glu Gly Asn Ser Ser Gln Asp Ser Ala Val Ala Ile
            580                 585                 590

Ala Thr His Val His Thr Glu Met His Arg Leu Leu Ala Ser Glu Gly
        595                 600                 605

Ala Asp Ala Gly Ser Gly Pro Glu Leu Leu Phe Tyr His Cys Glu Pro
    610                 615                 620

Pro Gly Ser Ala Val Leu Tyr Pro Phe Phe Leu Leu Asn Lys Gln Lys
625                 630                 635                 640

Thr Pro Ala Phe Glu His Phe Ile Lys Lys Phe Asn Ser Gly Gly Val
                645                 650                 655

Met Ala Ser Gln Glu Ile Val Ser Ala Thr Val Arg Leu Gln Thr Asp
            660                 665                 670

Pro Val Glu Tyr Leu Leu Glu Gln Leu Asn Asn Leu Thr Glu Thr Val
        675                 680                 685

Ser Pro Asn Thr Asp Val Arg Thr Tyr Ser Gly Lys Arg Asn Gly Ala
    690                 695                 700

Ser Asp Asp Leu Met Val Ala Val Ile Met Ala Ile Tyr Leu Ala Ala
705                 710                 715                 720

Gln Ala Gly Pro Pro His Thr Phe Ala Pro Ile Thr Arg Val Ser
                725                 730                 735

(2) INFORMATION FOR SEQ ID NO: 2:

    (i) SEQUENCE CHARACTERISTICS:
        (A) LENGTH: 1374 amino acids
        (B) TYPE: amino acid
        (C) STRANDEDNESS:
        (D) TOPOLOGY: unknown

    (ii) MOLECULE TYPE: protein

    (xi) SEQUENCE DESCRIPTION: SEQ ID NO: 2:

Met Ala Ala Pro Asn Arg Asp Pro Pro Gly Tyr Arg Tyr Ala Ala Ala
1               5                   10                  15

Met Val Pro Thr Gly Ser Leu Leu Ser Thr Ile Glu Val Ala Ser His
            20                  25                  30

Arg Arg Leu Phe Asp Phe Phe Ser Arg Val Arg Ser Asp Ala Asn Ser
        35                  40                  45

Leu Tyr Asp Val Glu Phe Asp Ala Leu Leu Gly Ser Tyr Cys Asn Thr
    50                  55                  60

Leu Ser Leu Val Arg Phe Leu Glu Leu Gly Leu Ser Val Ala Cys Val
65                  70                  75                  80

Cys Thr Lys Phe Pro Glu Leu Ala Tyr Met Asn Glu Gly Arg Val Gln
                85                  90                  95

Phe Glu Val His Gln Pro Leu Ile Ala Arg Asp Gly Pro His Pro Ile
            100                 105                 110

Glu Gln Pro Thr His Asn Tyr Met Thr Lys Ile Ile Asp Arg Arg Ala
        115                 120                 125

Leu Asn Ala Ala Phe Ser Leu Ala Thr Glu Ala Ile Ala Leu Leu Thr
    130                 135                 140

Gly Glu Ala Leu Asp Gly Thr Gly Ile Gly Ala His Arg Gln Leu Arg
145                 150                 155                 160

Ala Ile Gln Gln Leu Ala Arg Asn Val Gln Ala Val Leu Gly Ala Phe
                165                 170                 175

Glu Arg Gly Thr Ala Asp Gln Met Leu His Val Leu Leu Glu Lys Ala
            180                 185                 190

Pro Pro Leu Ala Leu Leu Leu Pro Met Gln Arg Tyr Leu Asp Asn Gly
        195                 200                 205

Arg Leu Ala Thr Arg Val Ala Arg Ala Thr Leu Val Ala Glu Leu Lys
    210                 215                 220

Arg Ser Phe Cys Glu Thr Ser Phe Phe Leu Gly Lys Ala Gly His Arg
225                 230                 235                 240

Arg Glu Ala Val Glu Ala Trp Leu Val Asp Leu Thr Thr Ala Thr Gln
                245                 250                 255

Pro Ser Val Ala Val Pro Arg Leu Thr His Ala Asp Thr Arg Gly Arg
            260                 265                 270

Pro Val Asp Gly Val Leu Val Thr Thr Ala Pro Ile Lys Gln Arg Leu
        275                 280                 285

Leu Gln Ser Phe Leu Lys Val Glu Asp Thr Glu Ala Asp Val Pro Val
    290                 295                 300

Thr Tyr Gly Glu Met Val Leu Asn Gly Ala Asn Leu Val Thr Ala Leu
305                 310                 315                 320

Val Met Gly Lys Ala Val Arg Ser Leu Asp Asp Val Gly Arg His Leu
                325                 330                 335

Leu Glu Met Gln Glu Glu Gln Leu Asp Leu Asn Arg Gln Thr Leu Asp

            340                 345                 350

Glu Leu Glu Ser Ala Pro Gln Thr Thr Arg Val Arg Ala Asp Leu Val
        355                 360                 365

Ser Ile Gly Glu Lys Leu Val Phe Leu Glu Ala Leu Glu Lys Arg Ile
    370                 375                 380

Tyr Ala Ala Thr Asn Val Pro Tyr Pro Leu Val Gly Ala Met Asp Leu
385                 390                 395                 400

Thr Phe Val Leu Pro Leu Gly Leu Phe Asn Pro Val Met Glu Arg Phe
                405                 410                 415

Ala Ala His Ala Gly Asp Leu Val Pro Ala Pro Gly His Pro Asp Pro
            420                 425                 430

Arg Ala Phe Pro Pro Arg Gln Leu Phe Phe Trp Gly Lys Asp Arg Gln
        435                 440                 445

Val Leu Arg Leu Ser Leu Glu His Ala Ile Gly Thr Val Cys His Pro
    450                 455                 460

Ser Leu Met Asn Val Asp Ala Ala Val Gly Gly Leu Asn Arg Asp Pro
465                 470                 475                 480

Val Glu Ala Ala Asn Pro Tyr Gly Ala Tyr Val Ala Ala Pro Ala Gly
                485                 490                 495

Pro Ala Ala Asp Met Gln Gln Leu Phe Leu Asn Ala Trp Gly Gln Arg
            500                 505                 510

Leu Ala His Gly Arg Val Arg Trp Val Ala Glu Gly Gln Met Thr Pro
        515                 520                 525

Glu Gln Phe Met Gln Pro Asp Asn Ala Asn Leu Ala Leu Glu Leu His
    530                 535                 540

Pro Ala Phe Asp Phe Phe Val Gly Val Ala Asp Val Glu Leu Pro Gly
545                 550                 555                 560

Gly Asp Val Pro Pro Ala Gly Pro Gly Glu Ile Gln Ala Thr Trp Arg
                565                 570                 575

Val Val Asn Gly Asn Leu Pro Leu Ala Leu Cys Pro Ala Ala Phe Arg
            580                 585                 590

Asp Ala Arg Gly Leu Glu Leu Gly Val Gly Arg His Ala Met Ala Pro
        595                 600                 605

Ala Thr Ile Ala Ala Val Arg Gly Ala Phe Asp Asp Arg Asn Tyr Pro
    610                 615                 620

Ala Val Phe Tyr Leu Leu Gln Ala Ala Ile His Gly Ser Glu His Val
625                 630                 635                 640

Phe Cys Ala Leu Ala Arg Leu Val Val Gln Cys Ile Thr Ser Tyr Trp
                645                 650                 655

Asn Asn Thr Arg Cys Ala Ala Phe Val Asn Asp Tyr Ser Leu Val Ser
            660                 665                 670

Tyr Val Val Thr Tyr Leu Gly Gly Asp Leu Pro Glu Glu Cys Met Ala
        675                 680                 685

Val Tyr Arg Asp Leu Val Ala His Val Glu Ala Leu Ala Gln Leu Val
    690                 695                 700

Asp Asp Phe Thr Leu Thr Gly Pro Glu Leu Gly Gly Gln Ala Gln Ala
705                 710                 715                 720

Glu Leu Asn His Leu Met Arg Asp Pro Ala Leu Leu Pro Pro Leu Val
                725                 730                 735

Trp Asp Cys Asp Ala Leu Met Arg Arg Ala Ala Leu Asp Arg His Arg
            740                 745                 750

Asp Cys Arg Val Ser Ala Gly Gly His Asp Pro Val Tyr Ala Ala Ala
        755                 760                 765

Cys Asn Val Ala Thr Ala Asp Phe Asn Arg Asn Asp Gly Gln Leu Leu
    770                 775                 780

His Asn Thr Gln Ala Arg Ala Ala Asp Ala Ala Asp Asp Arg Pro His
785                 790                 795                 800

Arg Gly Ala Asp Trp Thr Val His His Lys Ile Tyr Tyr Tyr Val Met
                805                 810                 815

Val Pro Ala Phe Ser Arg Gly Arg Cys Cys Thr Ala Gly Val Arg Phe
            820                 825                 830

Asp Arg Val Tyr Ala Thr Leu Gln Asn Met Val Val Pro Glu Ile Ala
        835                 840                 845

Pro Gly Glu Glu Cys Pro Ser Asp Pro Val Thr Asp Pro Ala His Pro
    850                 855                 860

Leu His Pro Ala Asn Leu Val Ala Asn Thr Val Asn Ala Met Phe His
865                 870                 875                 880

Asn Gly Arg Val Val Val Asp Gly Pro Ala Met Leu Thr Leu Gln Val
                885                 890                 895

Leu Ala His Asn Met Ala Glu Arg Thr Thr Ala Leu Leu Cys Ser Ala
            900                 905                 910

Ala Pro Asp Ala Gly Ala Asn Thr Ala Ser Thr Thr Asn Met Arg Ile
        915                 920                 925

Phe Asp Gly Ala Leu His Ala Gly Ile Leu Leu Met Ala Pro Gln His
    930                 935                 940

Leu Asp His Thr Ile Gln Asn Gly Asp Tyr Phe Tyr Pro Leu Pro Val
945                 950                 955                 960

His Ala Leu Phe Ala Gly Ala Asp His Val Ala Asn Ala Pro Asn Phe
                965                 970                 975

Pro Pro Ala Leu Arg Asp Leu Ser Arg Gln Val Pro Leu Val Pro Pro
            980                 985                 990

Ala Leu Gly Ala Asn Tyr Phe Ser Ser Ile Arg Gln Pro Val Val Gln
        995                 1000                1005

His Val Arg Glu Ser Ala Ala Gly Glu Asn Ala Leu Thr Tyr Ala Leu
    1010                1015                1020

Met Ala Gly Tyr Phe Lys Ile Ser Pro Val Ala Leu His His Gln Leu
1025                1030                1035                1040

Lys Thr Gly Leu His Pro Gly Phe Gly Phe Thr Val Val Arg Gln Asp
                1045                1050                1055

Arg Phe Val Thr Glu Asn Val Leu Phe Ser Glu Arg Ala Ser Glu Ala
            1060                1065                1070

Tyr Phe Leu Gly Gln Leu Gln Val Ala Arg His Glu Thr Gly Gly Gly
        1075                1080                1085

Val Asn Phe Thr Leu Thr Gln Pro Arg Ala Asn Val Asp Leu Gly Val
    1090                1095                1100

Gly Tyr Thr Ala Val Val Ala Thr Ala Thr Val Arg Asn Pro Val Thr
1105                1110                1115                1120

Asp Met Gly Asn Leu Pro Gln Asn Phe Tyr Leu Gly Arg Gly Ala Pro
                1125                1130                1135

Pro Leu Leu Asp Asn Ala Ala Ala Val Tyr Leu Arg Asn Ala Val Val
            1140                1145                1150

Ala Gly Asn Arg Leu Gly Pro Ala Gln Pro Val Pro Val Phe Gly Cys
        1155                1160                1165

Ala Gln Val Pro Arg Arg Ala Gly Met Asp His Gly Gln Asp Ala Val
    1170                1175                1180

Cys Glu Phe Ile Ala Thr Pro Val Ser Thr Asp Val Asn Tyr Phe Arg
1185                1190                1195                1200

Arg Pro Cys Asn Pro Arg Gly Arg Ala Ala Gly Gly Val Tyr Ala Gly
                1205                1210                1215

Asp Lys Glu Gly Asp Val Thr Ala Leu Met Tyr Asp His Gly Gln Ser

1220                1225                1230

Asp Pro Ser Arg Ala Phe Ala Ala Thr Ala Asn Pro Trp Ala Ser Gln
    1235                1240                1245

Arg Phe Ser Tyr Gly Asp Leu Leu Tyr Asn Gly Ala Tyr His Leu Asn
  1250                1255                1260

Gly Ala Ser Pro Val Leu Ser Pro Cys Phe Lys Phe Phe Thr Ser Ala
1265                1270                1275                1280

Asp Ile Ala Ala Lys His Arg Cys Leu Glu Arg Leu Ile Val Glu Thr
                1285                1290                1295

Gly Ser Ala Val Ser Thr Ala Thr Ala Ala Ser Asp Val Gln Phe Lys
            1300                1305                1310

Arg Pro Pro Gly Cys Arg Glu Leu Val Glu Asp Pro Cys Gly Leu Phe
    1315                1320                1325

Gln Glu Ala Tyr Pro Leu Thr Cys Ala Ser Asp Pro Ala Leu Leu Arg
  1330                1335                1340

Ser Ala Arg Asn Gly Glu Ala His Ala Arg Glu Thr His Phe Ala Gln
1345                1350                1355                1360

Tyr Leu Val Tyr Asp Ala Ser Pro Leu Lys Gly Leu Ala Leu
                1365                1370

## Description

### Field of the Invention

This invention relates generally to the field of anti-viral agents, and particularly to methods of identifying anti-viral agents active against members of the herpes family.

### Background of the Invention

Members of the Herpesviridae family include the human pathogens herpes simplex virus types 1 and 2 (HSV-1 and HSV-2), varicella-zoster virus (VZV), human cytomegalovirus (HCMV), human herpes viruses 6 and 7 (HHV-6 and HHV-7), Epstein-Barr virus (EBV) [reviewed in The Human Herpesviruses, ed. B. Roizman et al, Raven Press, NY (1993), pp. 1-9], and the Kaposi-sarcoma herpesvirus (KSHV) or HHV-8 [P. S. Moore et al, J. Virol., 70: 549-558 (1996)]. These viruses all have a large (approximately 100-270 kilobase pairs), linear, double-stranded DNA genome [reviewed in D. J. McGeoch, Annu. Rev. Microbiol., 43:235-265 (1989)]. During productive infection, viral DNA replication occurs via a proposed rolling circle mechanism producing concatameric copies of the viral genome. During maturation of the virion particle, a capsid particle self assembles, and the newly replicated viral DNA is cleaved into a unit length genome and subsequently packaged into the empty capsid (B-capsid) [See, The Human Herpesviruses, cited above, at pp. 11-68]. The processes of DNA cleavage and packaging of the viral DNA into the capsid are believed to be tightly coupled, as no viral mutants have yet been identified which cleave, but do not package DNA. Furthermore, this process of DNA cleavage and packaging is dependent upon the formation of an empty capsid, as mutations which prevent capsid assembly or maturation do not cleave the newly replicated concatameric viral DNA [reviewed in L. A. Tengelsen et al, J. Virol., 67:3470-3480 (1993)]. Although it is known which cis-acting sequences in the viral DNA are required for cleavage and packaging, the viral proteins responsible for this cleavage/packaging event and their exact functions in this event have not been well characterized. Most of the information regarding the genes involved in this process has come from studies using temperature sensitive or engineered mutations in HSV-1. Along with the six genes required for capsid assembly (UL18, UL19, UL26, UL26.5, UL35, and UL38) reviewed in D. R. Thomsen et al, J. Virol., 68:2442-2457 (1994), six other viral genes, UL6 [C. Addison et al, Virol., 138:246-259 (1984)], UL15 [C. Addison et al, J. Gen. Virol., 71:2377-2384 (1990)], UL25 [M.F. Al-Kobaisi et al, Virol., 180:380-388 (1991)], UL28 [G. Sherman and S.L. Bachenheimer, Virol., 158:427-434 (1987)], UL32 [G. Sherman and S.L. Bachenheimer, Virol., 163:471-480 (1988)], and UL33 [Y. Chang, et al., J. Virol., 70:3938-3946 (1996)], have been shown to be essential for DNA cleavage and packaging.

The genomic sequences for UL15 and UL19, as well as the sequences for the UL15 and UL19 gene products are available from GenBank under Accession No. 14112 (D00317). Further, the UL15 and UL19 sequences have been published [J. Baines et al, J. Virol., 68(12):8118-8124 (Dec. 1994) and Perry and Mcgeoch, J. Gen. Virol., 69:2831-2846 (1988), respectively]. The protein product of UL19 is VP5, also known as the major capsid protein.

Current methods for treating herpes virus infections have proved inadequate to prevent recurrence and viral shedding. Further, several of these treatments are toxic and are minimally effective.

What is needed are methods of identifying agents useful in treating and preventing infection with members of the herpesvirus family.

### Summary of the Invention

In one aspect, the present invention provides a method for identifying an antiviral agent useful in treating infection with herpes viruses. The method involves allowing HSV-1 UL15 or a functional derivative or homologue thereof to come into association with a test compound. HSV-1 VP5 or a functional derivative or homologue thereof is then allowed to come into association with the HSV-1 UL15 or its derivative or homologue. Inhibition of the interaction between UL15 or its derivative or homologue and VP5 or its derivative or homologue is then determined, wherein said inhibition is indicative that the test compound is an antiviral agent.

In another aspect, the present invention provides a method for identifying an antiviral agent useful in treating infection with herpes viruses. The method involves allowing HSV-1 VP5 or a functional derivative or homologue thereof to come into association with a test compound. HSV-1 UL15 or a functional derivative or homologue thereof is then allowed to come into association with the HSV-1 VP5 or its derivative or homologue. Inhibition of the interaction between VP5 or its derivative or homologue and UL15 or its derivative or homologue is then determined, wherein said inhibition is indicative that the test compound is an antiviral agent.

In yet another aspect, the present invention provides a method for identifying antiviral agents which inhibit the function of the UL15/VP5 complex. This method involves allowing a sample containing a test compound to come into association with a herpes-infected cell and screening for viral inhibition. A test compound so identified is then screened for the ability to block the function of a complex comprising UL15 or a functional derivative or homologue thereof and VP5 or a functional derivative or homologue thereof.

In a further aspect, the present invention provides compounds identified by the above methods.

Other aspects and advantages of the present invention are described further in the following detailed description of the preferred embodiments thereof.

### Detailed Description of the Invention

The present invention provides a method for identifying antiviral agents, particularly agents useful in preventing and treating infection with members of the herpes family, and compounds identified by the method of the invention. The method of the invention involves identifying molecules which prevent UL15/VP5 interaction and/or function.

For convenience, the amino acid sequences of UL15 and VP5 as provided in GenBank are reproduced in SEQ ID NO: 1 and SEQ ID NO:2, respectively. However, the present invention is not limited to the use of these amino acids sequences, as described below.

BINDING MOLECULE, as used herein, refers to molecules or ions which bind or interact specifically with UL15 protein or functional derivatives or homologues thereof and/or, bind or interact specifically with VP5 or functional derivatives or homologues thereof, and/or bind or interact with the complex formed by UL15/VP5 (or functional derivatives or homologues thereof). Such binding molecules include, for example, small organic molecules, peptides, polypeptides, antibodies, particularly intracellular antibodies, antibody proteins and other antibody-derived reagents, chemical agents, nucleic acid sequences, enzyme substrates, cell membrane components and classical receptors. Interaction between the UL15 and/or UL19 gene products and such binding molecules, is indicative of anti-viral activity of the binding molecule. Prior to identification of a molecule as a binding molecule through the method of the invention, the molecule is referred to as a test compound.

As used herein, the term "FUNCTIONAL DERIVATIVE" of UL15 or VP5 includes fragments of these proteins, including peptides. Desirably, a UL15 peptide is characterized by the ability to form a complex with VP5 or a functional derivative or homologue thereof (e.g., by binding or otherwise interacting), and a VP5 peptide is characterized by the ability to form a complex with UL15 or a functional derivative or homologue thereof. For example, suitable fragments include, but are not limited to, fragments of UL15 located between about amino acid residue 1 to about amino acid residue 409 of SEQ ID NO:1, and between about residues 1 to about residues 508 of UL15 of SEQ ID NO:1. Other suitable UL15 proteins and peptides include those encoded by other splice variants of the UL15 gene. Similarly, included within this definition are homologues and/or peptides (i.e., fragments) of VP5. Functional derivatives also include UL15 and VP5 proteins and peptides which have been fused to or otherwise provided with a tag, e.g., an epitope tag, a polyHis tag, or the like, or a selectable marker or other detection means, for use in the various methods described herein. Suitable tags, markers and other means of detection are well known and can be readily selected by one of skill in the art.

Further included within the definition of functional derivatives are fusion proteins containing these UL15 or VP5 proteins or, preferably, peptides fused directly or indirectly (i.e. via linkers) to a fusion partner. Linkers and fusion partners for the peptides may be chosen for convenience in expression and/or purification. Suitable linkers may be readily selected by one of skill in the art. Similarly, suitable fusion partners, e.g., GST, Ga14, lexA, maltose-binding protein (MBP), lacZ, trp, and the like, may be readily selected by one of skill in the art. Also included within the definition of functional derivatives are UL15 and VP5 proteins which have been modified for ease of expression and/or purification.

For convenience throughout this application, the terms UL15 and VP5 will be used. However, it should be understood that one could readily substitute a functional derivative or homologue of either or both of these proteins in the methods of the invention.

Without wishing to be bound by theory, the inventors have found that the UL15 gene product specifically interacts with the major capsid protein VP5 (the gene product of UL19). As shown herein, experiments employing UL15 and VP5 proteins expressed using recombinant baculoviruses show that these two viral proteins are sufficient for this interaction. Furthermore, sequences contained within the amino terminus of UL15 appear to be critical for this interaction. This discovery, coupled with the knowledge that the UL15 gene and its homologues are the most highly conserved amongst all members of the *Herpesviridae,* permitted the inventors to develop the method of the invention. For convenience, the specification will refer to UL19 and VP5. However, as discussed, the methods described herein may be readily performed with functional derivatives or homologues of HSV-1 UL15 in other human herpesviruses, which include, but are not limited to, ORF42/45 in VZV, UL89 in HCMV, BD/BGRF1 in EBV, and NCBIgi 325496 in HHV-6, or suitable non-human herpesviruses. Similar methods can be performed with functional derivatives of UL19 or UL19 homologues derived from human or suitable non-human herpesviruses.

Thus, the present invention provides methods for screening and identifying agents which are active as anti-viral compounds for members of herpesviridae. Because UL15 and VP5 and their homologues are the most highly conserved proteins among the herpesvirus family, it is anticipated that an anti-viral agent identified by the method of the invention will be useful in prevention and treatment of a, b and g herpes viruses. However, any selected anti-viral agent of the invention may exhibit greater activity against viruses in one of these subgroups in comparison to its activity against viruses from another subgroup.

Desirably, the method of the invention involves the use of assays which detect or measure protein-protein associations. Particularly, the assays detect a molecule which binds to and occupies the binding site of UL15 thereby preventing binding to VP5, such that normal viral activity is prevented. Similarly, assays which detect a molecule which binds to and occupies the binding site of VP5 thereby preventing binding to UL15 and prevent normal viral activity, can be used. In another alternative, an assay may identify a molecule which inhibits the function of a UL15/VP5 complex. In yet another alternative, the assays may detect a molecule which binds to a site on UL15 or VP5 different from the normal binding site, which nevertheless inhibits the ability of UL15 and VP5 to interact and function normally, via allosteric effect.

Particularly, test compounds which interfere with the interaction between UL15 and VP5, or interfere with their function, have been found by the inventors to be useful as anti-viral agents for treatment and prophylaxis of herpes family viral infections. Most desirably, the test compound interacts with UL15 (or VP5) and inhibits its interaction with VP5 (or UL15). Thus, UL15 and VP5 can be used to identify and assess the binding of small molecule substrates and ligands in, for example, cells, cell-free preparations, chemical libraries, and natural product mixtures. These substrates and ligands may be natural substrates and ligands; may be structural or functional mimetics; or may be unrelated to the natural binding events. For example, the method of the invention may involve employing a suitable computer program to determine compounds having structures complementary to that of UL15, VP5, or portions thereof and screening those compounds for competitive binding to the protein.

To identify small molecule antagonists, a rapid and reproducible assay is desirable for the screening of synthetic compounds, natural products, and peptides. Such an assay is desirably a protein based assay which would isolate UL15 and/or VP5 from interference by other assay components, particularly when utilizing cell-based assays. Desirably, the assay is adaptable to automation. Such assays are well known to those of skill in the art and are not intended to be a limitation on the present invention.

Where desired, and depending on the assay selected, UL15 (or, in an alternative embodiment, VP5) may be immobilized on a suitable surface. Such immobilization surfaces are well known. For example, a wettable inert bead may be used in order to facilitate multivalent interaction with UL15 (or VP5). Alternatively, an antibody to UL15 (or VP5) may be used. Typically, the surface containing UL15 (or VP5) is permitted to come into contact with a solution containing the test compound and a solution containing VP5 (or UL15) and binding is measured using an appropriate detection system. Suitable detection systems include the streptavidin horse radish peroxidase conjugate, direct conjugation by a tag, e.g., fluorescein. Other systems are well known to those of skill in the art. This invention is not limited by the detection system used.

Further, the methods of the invention are readily adaptable to combinatorial technology, where multiple molecules are contained on an immobilized support system. In such a method, a solution containing UL15 (or, in an alternative embodiment, VP5) is provided to the support system, followed by a solution containing VP5 (or UL15). Thus, the method of the invention permits screening of chemical compound and peptide based libraries.

The assay methods described herein are useful in screening for inhibition of the interaction between UL15 and VP5. In a preferred method, a solution containing the suspected inhibitors (i.e., the test compounds) is contacted with immobilized UL15 (or, in an alternative embodiment, VP5) substantially simultaneously with contacting the immobilized UL15 (or immobilized VP5) with the solution containing VP5 (or UL15). The solution containing the inhibitors may be obtained from any appropriate source, including, for example, extracts of supernatants from culture of bioorganisms, extracts from organisms collected from natural sources, chemical compounds, and mixtures thereof. In another variation, the inhibitor solution may be added prior to or after addition of VP5 (or UL15) to the immobilized UL15 (or VP5). As stated above, similar methods may be performed using homologues to UL15 or VP5, such as those identified above.

One example of a suitable assay is the yeast two-hybrid system. The yeast two-hybrid system provides methods for detecting the interaction between a first test protein (i.e., UL15 or vice-versa) and a second test protein (i.e., VP5 or vice-versa) using reconstitution of the activity of a transcriptional activator. The method is disclosed in U.S. Patent No.5,283,173; reagents are available from Clontech and Stratagene. Briefly, UL15 (or UL19) cDNA is fused to a Ga14 transcription factor DNA binding domain and expressed in yeast cells. Conversely, UL15 (or UL19) is fused to a transactivation domain of Ga14. Interaction of the fusion proteins via the UL15 and VP5 domains will lead to reconstitution of Ga14 activity and transactivation of expression of a reporter gene such as Ga11-lacZ.

As another example of a suitable assay, an enzyme-linked immunoassay (ELISA) or another immunoassay format may be utilized in which UL15 (or, in an alternative embodiment, VP5) is immobilized, directly or indirectly to a suitable surface. Such a immunoassay will typically involve use of specific antibodies (e.g., anti-UL15 or anti-VP5) or incubation of radiolabeled UL15 or VP5 with filter separation steps. In the presence of test substances which interrupt or inhibit formation of a UL15/VP5 complex, an increased amount of free UL15 or VP5 will be determined relative to a control lacking the test substance.

Yet another alternative method involves screening of peptide libraries for binding partners. Recombinant tagged or labeled UL15 (or, in an alternative embodiment, VP5) is used to select peptides from a peptide or phosphopeptide library which interact with the UL15 (or, in an alternative embodiment, VP5). Sequencing of the peptides leads to identification of consensus peptide sequences which might be found in interacting proteins.

Another method is immunoaffinity purification. Recombinant UL15 (or, alternatively VP5) is incubated with labeled or unlabeled cell extracts and immunoprecipitated with anti-UL15 (or anti-VP5) antibodies. The immunoprecipitate is recovered with protein A-Sepharose and analyzed by SDS-PAGE. Unlabelled proteins are labeled by biotinylation and detected on SDS gels with streptavidin. The binding molecules are then analyzed for inhibition of the UL15/VP5 interaction.

This assessment of anti-viral activity of the test compounds identified as binding molecules can be performed using standard assays. Such assays will be readily apparent to one of skill in the art. Examples of such assays include, without limitation, plaque reduction assays and virus yield reduction assays.

In yet another aspect of the invention, methods for identifying antiviral agents by screening for inhibition of the function of the UL15/VP5 complex are provided. Suitable assays for performance of these methods are well known to those of skill in the art. For example, a cell-free system containing the UL15/VP5 complex may be utilized.

More desirably, however, cell-based assays are utilized in these screens. For example, the host cells may be modified to transiently or stably express UL15 and/or VP5. Alternatively, in a currently preferred embodiment, such an assay utilizes a cell infected with a herpes virus. A sample containing one or more test compounds is then brought into association with the infected cell and inhibition of normal herpes viral function is detected using methods such as those described herein and which are well known in the art. The test compound(s) identified are then screened for the ability to inhibit a complex comprising UL15 and VP5. This inhibition may be indirect, e.g., the compound may inhibit the function of the complex, or direct, e.g., by blocking binding of UL15 and VP5.

Test compounds identified by the methods described herein, which directly or indirectly (e.g., functionally) inhibit UL15/VP5 may be assayed using standard techniques.

### Compositions

The present invention further provides compounds identified by the methods of the invention and the use of the compounds as anti-viral agents useful in pharmaceutical compositions for the prophylaxis or treatment of infection with a member of the herpesvirus family.

The anti-viral agents identified by the method of the invention may be formulated into pharmaceutical compositions which may be used prophylactically or therapeutically, and preferably post-infection, to treat a herpes viral infection. The amount employed of the binding molecule/anti-viral agent will vary with the manner of administration, the employment of other active compounds, and the like, generally being in the range of about 1 mg to 10 mg, formulated in a solution of about 0.5 ml to 5 ml of a suitable carrier. Thus, the anti-viral agents of the present invention may be employed in combination with a non-sterile or sterile carrier or carriers for use with cells, tissues or organisms, such as a pharmaceutical carrier suitable for administration to a subject. Such compositions comprise, for instance, a media additive or a therapeutically effective amount of an anti-viral agent of the invention and a pharmaceutically acceptable carrier or excipient. Such carriers may include, but are not limited to, saline, buffered saline, dextrose, water, glycerol, ethanol and combinations thereof. The formulation should suit the mode of administration.

The pharmaceutical compositions may be administered in any effective, convenient manner including, for instance, administration by topical, oral, anal, vaginal, intravenous, intraperitoneal, intramuscular, subcutaneous, intranasal or intradermal routes, among others.

These examples are illustrative only and do not limit the scope of the invention.

### Example 1 - Detection of UL15 Gene Product/VP5 Complex

UL15 was immunoprecipitated from HSV-1 infected Vero cells [ATCC, CCL81], and the immunoprecipitate was immunoblotted with antisera to the various capsid proteins. More particularly, Vero cells were infected with 10 PFU per cell of HSV-1 (KOS) [K. O. Smith, Proc. Soc. Exp. Biol. Med., 115:814-816 (1964); P. A. Schaffer et al, Virology, 52:57-71 (1973)] and were harvested 20 hours post infection. Cells were lysed by freeze-thawing three times in phosphate buffered saline (PBS) followed by centrifugation to pellet debris. The supernatant was pre-cleared with normal rabbit serum and protein-A bound to sepharose. The resulting supernatant was immunoprecipitated with five microliters of UL15-specific antisera (NC-1) [J. D. Baines, J. Virol., 68(12):8118-8124 (December 1994)], immunoblotted, and probed with NC-1 antisera to the HSV-1 capsid proteins [G. H. Cohen et al, J. Virol., 34(2):521-531 (May 1980)].

The UL15 polyclonal antisera directed against the carboxy-terminal portion of the UL15 protein that was used has been previously shown to react with two proteins of 75 kD and 35 kD molecular weight [Baines et al, cited above]. Immunoprecipitates of UL15 from HSV-1 infected Vero cells were also found to contain a 155 kD protein which immunoreacts with antisera against the major capsid protein VP5 and corresponds with the reported size of VP5. This band was not present in uninfected Vero cells. Conversely, a band of 75 kD which was immunoreactive with antisera against UL15 was detected when VP5 was immuno-precipitated from HSV-1 infected Vero cells, but not uninfected Vero cells. Furthermore, none ofthe 35 kD UL15 protein was detected in the VP5 immunoprecipitates, nor was any UL15 5 protein detected in a preparation of sucrose-density banded, purified HSV-1 capsids, suggesting that UL15 is not a capsid protein. Probing of immunoblots of UL15 immuno-precipitates with antisera to the other HSV- I capsid proteins did not reveal co-immuno-precipitation with these other proteins. This could be due to either a weak interaction which was not detectable by this method, or by the low abundance of the capsid protein in the UL15 immunoprecipitate, as several capsid genes, such as VP21 and VP24, are not present in great amounts within the capsid. The data indicates that in HSV-1 infected cell extracts, UL15 and VP5 complex together.

### Example 2 - Characterization of UL15/VP5 Complex

To determine if the UL15/VP5-containing complex identified in Example 1 contains any other proteins, whether UL15 and VP5 directly interact within the complex, or whether only UL15 and VP5 are sufficient for complex formation, the following study was performed.

UL15 and VP5 were expressed recombinantly using a baculovirus expression system. The open reading frames (ORFs) of the UL15 (VP5) gene was derived as partial cDNA from Vero cells infected form HSV-1 (KOS) and pSG10 and pSG16 plasmids containing the gene [described in A. L. Goldin et al, J. Virol., 38(10):50-58 (April 1981)]. The UL19 ORF was similarly derived, using plasmid pSG16 [Goldin et al, cited above]. The UL15 and UL19 ORFs were cloned into the baculovirus expression vector pVL 1393 [Pharminogen] and used to generate recombinant baculoviruses. Susceptible Sf9 cells [ATCC] were co-infected with these two recombinant baculoviruses (BAC-UL 15 and BAC-VP5) at a multiplicity-of-infection (MOI) of 5 PFU of each virus per cell. Total protein lysates were prepared at 48 hours by direct lysis in I ml of sample buffer. Uninfected Sf9 cells, cells infected wild type baculovirus, cells infected with wild-type baculovirus and BAC-UL15, and cells infected with wild type baculovirus and BAC-VP5 were used as controls. Twenty-five microliters of this sample was loaded onto a 4-20% gradient polyacrylamide gel containing 0.1% SDS, and electrophoresed in Tris-glycine buffer. Following electrophoresis and electrotransfer, the gel was immunoblotted and probed with rabbit polyclonal antisera specific for either VP5 (NC1) (using UL15 antisera) or UL15 (using VP5 antisera).

Specifically immunoreactive bands of the appropriate sizes (75 kD for UL15) and (155 kD for VP5) were observed in cells infected with the respective baculovirus and not in uninfected Sf9 cells or Sf9 cells infected with wild type baculovirus.

### Example 3 - Specificity of UL15:VP5 Complex

Because VP5 is a rather large protein (155 kD), it was important to determine if UL15:VP5 complex formation was due to a non-specific sticking of the two proteins. To address this question, UL15 and several other unrelated polypeptides were synthesized *in vitro* by coupled transcription/translation and tested for their ability to interact with VP5 expressed by baculovirus as follows.

Template DNAs containing the open reading frames of UL15, and negative controls HSV-1 UL12 (alkaline exonuclease), human papillomavirus (HPV) type 6b E1 (HPV-6b E1), HPV-16 E1, HPV-18 E2, and HPV-16 E2 proteins were used to synthesize their respective radiolabeled proteins in vitro in a 50 microliter synthesis reaction. A small portion of the reaction (1/10th) was analyzed by SDS-PAGE to confirm the expression of these proteins. To determine if VP5 interacted with these proteins, the remainder of the reaction was mixed with 50 ml of an extract made from BAC-VP5 infected Sf9 cells, immunoprecipitated with VP5 antisera, and analyzed by SDS-PAGE.

Although a background amount of HPV-6b E1, HPV-16 E1, and HPV-16 E2 proteins co-immunoprecipitated with VP5, approximately a 10-fold greater amount of UL15 was co-immunprecipitated with VP5, strongly suggesting that the interaction of UL15 and VP5 was due to specific interaction with VP5.

### Example 4 - Identification of Region of UL15 Interacting with VP5

To determine which region of UL15 was involved in complex formation with VP5, a series of UL15 polypeptides which were truncated at the carboxy-terminus were synthesized *in vitro* by coupled transcription/translation and examined for their ability to interact with VP5.

Truncated UL15 proteins which contained amino acids 409-735 [SEQ ID NO:1] were still able to coimmunoprecipitate with VP5. However, UL15 proteins which further deleted amino acids between 1 - 409 [SEQ ID NO: 1] were unable to interact with VP5, suggesting that amino acids in the amino-terminus of UL15 are critical for VP5 interaction. This finding is also confirmed by the observation that the 35kD form of UL15 which lacks the amino-terminal of the 75 kD protein, but shares only the carboxy-terminal region does not coimmunoprecipitate with VP5.

The data presented in Examples 1-4 show that the 75 kD protein from the UL15 gene coimmunoprecipitates with the major capsid protein VP5. This interaction may provide a means for linking the terminase complex with the empty capsid so that the newly replicated DNA can be easily packaged. Inspection of the UL15 sequence reveals the presence of a potential cleavage site for the UL26 protease located at amino acids 332-339 [SEQ ID NO:1]. If this site represents a genuine substrate for the UL26 protease, it may explain the generation of the 35 kD protein, and also provide a means by which UL15 terminase activity could be inactivated signalling the completion of DNA packaging.

### Example 5 - Application of UL15 and VP5 proteins in a screen for molecules blocking the interaction of UL15 with VP5

The following assay format may be used to identify molecules which inhibit UL15:VP5 binding by screening a large bank of chemical compounds and natural products.

The VP5 protein is biotinylated for simplicity of assay and for ease of detection. Biotinylation is carried out essentially as described in Avidin-Biotin Chemistry: A handbook, M. D. Savage *et al.,* Pierce Chemical Company (1992). All steps of the assay after coating are carried out at room temperature.

The wells of 96 well microtiter plates (Immunlon 4, Dynatech Laboratories) are coated with UL15 5 gene product (2 mg/ml) in 100 ml/well of 0.1 M sodium bicarbonate, pH 9.4 and incubated overnight @ 4°C. Immediately afterwards, dilutions of test compound are added (10 ml). The compounds are dissolved at 100x assay concentration in dimethyl sulfoxide (DMSO) and subsequently diluted in 50%DMSO/50% H₂O to a 10X working stock.

The wells are washed with PBS (phosphate buffered saline) and blocked with 0.5% gelatin in PBS for 1 hour. Following an additional PBS wash, biotinylated VP5 is serially diluted in PBS containing 1 mg/ml BSA, 0.05% Tween directly in the wells in a final volume of 0.1 ml and incubated for 1 hour. The wells are washed with PBS and bound VP5 protein is measured by the addition of 0.1 ml of strepavidin-HRP (streptavidin conjugated with horseradish peroxidase (Southern Biotech)) at a 1:2000 dilution for 1 hour, followed by washing and color development with 100 ml ABTS substrate (Kierkegaard and Perry Laboratories Inc., Maryland) and measurement of absorbance at 405 nm. In some cases the color reactions are arrested by addition of 100 ml of 1% SDS prior to measurement of absorbance.

Numerous modifications and variations of the present invention are included in the above-identified specification and are expected to be obvious to one of skill in the art. Such modifications and alterations to the compositions and processes of the present invention are believed to be encompassed in the scope of the claims appended hereto.

## Claims

1. A method for identifying an antiviral agent useful in treating infection with herpes viruses,
(a) providing UL15 of herpes simplex virus-1 (HSV-1) or a functional derivative or homologue thereof;
(b) permitting UL15 or its derivative or homologue to come into association with a test compound;
(c) providing VP5 of HSV-1 or a functional derivative or homologue thereof;
(d) screening for inhibition of the interaction between UL15 or its derivative or homologue and VP5 or its derivative or homologue, wherein said inhibition is indicative that the test compound is an antiviral agent.

2. A method for identifying an antiviral agent useful in treating infection with herpes viruses,
(a) providing VP5 of herpes simplex virus-1 (HSV-1) or a functional derivative or homologue thereof;
(b) permitting VP5 or its derivative or homologue to come into association with a test compound;
(c) providing UL15 of HSV-1 or a functional derivative or homologue thereof;
(d) screening for inhibition of the interaction between the VP5 or its derivative or homologue and UL15 or its derivative or homologue, wherein said inhibition is indicative that the test compound is an antiviral agent.

3. The method according to claim 1 or 2 wherein a UL15/VP5 complex comprising UL15 or a functional derivative or homologue thereof and VP5 or a functional derivative or homologue thereof is permitted to come into association with a test compound.

4. The method according to claim 1 or 2 wherein the test compound binds the UL15/VP5 complex.

5. The method according to claim 1 or 2 wherein the test compound binds UL15 or a functional derivative or homologue thereof.

6. The method according to claim 1 or 2 wherein the test compound binds VP5 or a functional derivative or homologue thereof.

7. The method according to claim 1 or 2 wherein the functional derivative of UL15 is selected from the group consisting of:
(a) SEQ ID NO:1;
(b) a peptide from SEQ ID NO:1;
(c) a fusion protein comprising (a) or (b) and a selected fusion partner;
and
(d) a tagged protein comprising (a) or (b) and a selected marker or tag.

8. The method according to claim 7, wherein the UL15 peptide is selected from the group consisting of:
(a) about amino acid 1 to about amino acid 409 of SEQ ID NO:1;
and
(b) about amino acid 1 to about amino acid 508 of SEQ ID NO:1.

9. The method according to claim 7, wherein the fusion partner is selected from the group consisting of: GST, Ga14, lexA, maltose-binding protein, lacZ, and Trp.

10. The method according to claim 1 or 2, wherein the functional derivative of VP5 is selected from the group consisting of:
(a) SEQ ID NO:2;
(b) a peptide from SEQ ID NO:2;
(c) a fusion protein comprising (a) or (b) and a selected fusion partner.
(d) a tagged protein comprising (a) or (b) and a and a selected marker or epitope tag.

11. The method according to claim 1 or 2 wherein the UL15 homologue is selected from the group consisting of:
varicella-zoster virus ORF42/45;
human cytomegalovirus UL89;
epstein-barr virus BD/BGRF1; and
human herpes virus 6 in NCBIgi 325496.

12. The method according to claim 1 or 2 wherein UL15 is immobilized on a solid support.

13. The method according to claim 1 or 2 wherein VP5 is immobilized on a solid support.

14. The method according to claim 1 or 2 wherein said screen is a yeast 2-hybrid screen.

15. The method according to claim 1 or 2 wherein said screen is an enzyme-linked immunosorbent assay.

16. The method according to claim 1 or 2 wherein the test compound is immobilized on a support system.

17. The method according to claim 1 or 2, wherein said test compound is a chemical agent.

18. The method according to claim 1 or 2, wherein said test compound is a polypeptide.

19. The method according to claim 1 or 2, wherein said test compound is an intracellular antibody.

20. The method according to claim 1 or 2 wherein steps (b) and (c) occur substantially simultaneously.

21. A compound identified by the method according to claim 1 or 2.

22. A pharmaceutical composition comprising a compound according to claim 21 and a pharmaceutically acceptable carrier.

23. A method for identifying antiviral agents which functionally inhibit a UL15/VP5 complex comprising UL15 or a functional derivative or homologue thereof and VP5 or a functional derivative or homologue thereof, said method comprising the steps of:
(a) providing a cell infected with a herpes virus;
(b) permitting a sample containing a test compound to come into association with the herpes-infected cell;
(c) identifying a test compound which inhibits the herpes virus;
(d) screening the test compound which is identified in (c) for the ability to block the function of the UL15/VP5 complex.

24. A method for identifying antiviral agents which functionally inhibit a UL15/VP5 complex comprising UL15 or a functional derivative or homologue thereof and VP5 or a functional derivative or homologue thereof, said method comprising the steps of:
(a) providing a cell-free system containing the UL15/VP5 complex;
(b) permitting a sample containing a test compound to come into association with the complex; and
(c) identifying a test compound which inhibits the function of the UL15/VP5 complex.

25. A method for identifying antiviral agents which functionally inhibit a UL15/VP5 complex comprising UL15 or a functional derivative or homologue thereof and VP5 or a functional derivative or homologue thereof, said method comprising the steps of:
(a) providing a cell expressing UL15 and VP5;
(b) permitting a sample containing a test compound to come into association with the UL15-, VP5-expressing cell; and
(c) identifying a test compound which inhibits the function of the UL15/VP5 complex.

26. An antiviral agent identified by any of the methods of claim 23 - 25.
